# EUROPEAN PATENT APPLICATION

(11) **EP 2 397 491 A1**
(43) Date of publication of application: **21.12.2011**
(21) Application number: 10166661.8
(22) Date of filing: 21.06.2010
(51) Int. Cl.: C07K 14/37, C12R 1/885

(54) **LeaA from Trichoderma reesei**

(71) Applicant: Technische Universität Wien, 1040 Wien (AT)
(72) Inventor: Kubicek, Christian, 1100 Wien (AT); Seiboth, Bernhard, 1190 Wien (AT); Linke, Rita, 1120 Wien (AT); Karimi, Razieh, 1180 Wien (AT)
(74) Representative: Sonn & Partner Patentanwälte

(57) **Abstract**

The present invention relates to isolated polypeptide with transcription promoting protein methyltransferase activity having at least 80% identity with amino acid sequence SEQ ID No. 1 and uses thereof.

SEQUENCE LISTING

<110>  Technische Universität Wien

<120>  Polypeptide

<130>  R56216

<160>  11

<170>  PatentIn version 3.5

<210>  1
<211>  332
<212>  PRT
<213>  Trichoderma reesei

<400>  1

Met Ser Arg Asn Ala Pro Asn Gly Cys Val Pro Pro Ser Gln Ala Thr
1               5                   10                  15

Ala Pro Pro Ser Pro Ala Thr Ser Leu Arg Leu Thr Val Gly Glu Pro
            20                  25                  30

Val Ser Glu Pro Ala Thr Glu Ser Gly Glu Arg Val Leu Gln Asp Gly
        35                  40                  45

Phe Trp Glu His Gly Arg Phe Tyr Gly Ser Trp Lys Pro Gly Lys Tyr
    50                  55                  60

Leu Phe Pro Ile Asp Lys Glu Glu Leu Asn Arg Leu Asp Val Phe His
65                  70                  75                  80

Lys Tyr Phe Leu Val Ala Arg Asp Glu Lys Val Thr Ser Thr Pro Leu
                85                  90                  95

Arg Lys Asp Gly Arg Pro Lys Ile Met Asp Leu Gly Thr Gly Thr Gly
            100                 105                 110

Ile Trp Ala Tyr Asn Val Val Glu Glu Tyr Ala Lys Asp Ala Glu Ile
        115                 120                 125

Met Ala Val Asp Leu Asn Gln Ile Gln Pro Ala Leu Ile Pro Arg Gly
    130                 135                 140

Val Thr Thr Lys Gln Phe Asp Ile Glu Glu Pro Ser Trp Asp Pro Leu
145                 150                 155                 160

Leu Arg Asp Cys Glu Leu Ile His Met Arg Leu Leu Tyr Gly Ser Ile
                165                 170                 175

Arg Asp Asp Lys Trp Pro His Val Tyr Arg Lys Ala Phe Glu His Leu
            180                 185                 190

Ala Pro Gly Ile Gly Tyr Ile Glu Gln Leu Glu Ile Asp Trp Met Pro
        195                 200                 205

Arg Trp Glu Asn Glu Asp Leu Pro Arg His Ser Ala Leu Gln Glu Trp
    210                 215                 220

Ala Gln Leu Phe Gln Arg Ala Met His Arg Tyr His Arg Ser Val Thr
225                 230                 235                 240

Val Ser Gly Glu Ala Thr Arg Arg Arg Met Glu Ala Ala Gly Phe Thr
                245                 250                 255

Asp Phe Ser Glu Thr Thr Ile Arg Cys Tyr Val Asn Pro Trp Ser Pro
            260                 265                 270

Asp Arg His Gln Arg Glu Cys Ala Arg Trp Phe Asn Leu Ala Phe Ser
        275                 280                 285

Leu Gly Leu Glu Ala Met Ser Met Met Pro Met Ile Asp Lys Leu Gly
    290                 295                 300

Met Thr Lys Asp Asp Ile Val Asp Leu Cys Ser Arg Ala Lys Lys Glu
305                 310                 315                 320

Met Cys Ile Leu Arg Tyr Arg Ala Tyr Cys Thr Leu
                325                 330

<210>  2
<211>  780
<212>  DNA
<213>  Trichoderma reesei

<400>  2
atgtctcgaa acgctcccaa cgggtgtgtt ccaccctccc aagctactgc tccgccttcg        60

ccagccacaa gtctgcgact aacagttggg gaaccggtca gcgagccggc cactgaatcc       120

ggggagagag ttctccagga tgggttctgg gagcacggtc gcttttatgg ttcttggaag       180

cctgggaaat accttttccc catagacaag gaggagctca ataggttaga tgtctttcac       240

aagtatttcc tcgttgcaag agacgagaaa gtcacttcaa ctcccctgag gaaagatgga       300

cggccgaaaa tcatggatct cggcacaggc acgggcatct gggcgtataa tgttgtggaa       360

gagtatgcca aggatgccga aatcatggcc gtggatctca atcaaattca accagctctg       420

cacttggccc ctggcattgg ctatatcgag caactggaga ttgactggat gccgcgatgg       480

gagaatgagg atctccccag acattcggct cttcaagaat gggctcagct attccaacgt       540

gccatgcatc gctaccaccg cagcgtcacg gtatcaggcg aggctaccag acgcagaatg       600

gaagcggctg gctttacaga tttctccgaa acaacgatcc ggtgctacgt aaacccgtgg       660

tctcccgatc gccatcagcg ggagtgtgcc cgttggttca acctcgcctt cagcctcggc       720

cttgaggcca tgagcatgat gccaatgatt gacaaactcg gcatgaccaa ggacgatatc       780

<210>  3
<211>  4061
<212>  DNA
<213>  Trichoderma reesei

<400> 3
ccttttacca acttggcagc ccttgcctct tcgttgctgg ctagtagggg aggcaggcca 60
ttgatcccgg gctcgcgtca atccaccaag ccccaaagag ccctagaagc tcgcgacact 120
tgtcattgaa ccaacacgac tctcaaccgc cgtctgtcga ttctcacttc ggcattcgtc 180
gacctcctcc ctccagccgc tggtccactc cggacccgag cttcgcgcca gtcttaaagg 240
gctagccgtc ctcgcccccc cttctccagt ccgccgaacg acagtctcaa cctcaaccct 300
gggagtcgtg ataattttct ataccgccct tctccgctct ctcagccgtc actcgcttgt 360
gtcttccaac cggagctgtc cacgctgccg cctgggacga ttacgccctt gcctgtcgct 420
gcattcgtgc tgcaaattgg ttgctccggt ggctcaggtg accctggtcg aggacttcta 480
cctctttcaa ggagcctcgt gagagttgat acagcagctg ccctcgacag gtcacacaaa 540
cacatgggat cgtcgcttat tctgacctac ggcagctgcg cggcaaccca cccgtaccac 600
aggcatcggt atctgtgtca cctccttgac atctcggcca gttgaatttc tgatgtaagt 660
tgccatctgc cttgttgttg ttcgtctggc tgccctacac catcttgccc gcctgctcgg 720
ctcccatcta ctccttgatt tatctttcca tcttgtctta ctgccatgat gcgcgttgaa 780
aggcgccatg catgccatcg tgtccggcca ggatatgaat gctgtgctca gtcagccgtc 840
ttcctttttg gaagccaatc agcaaggttg aactgtcttt ctctttcctt gccatcatca 900
acccttgcgg cagatgttta tgtctctcgc cccttgcctt gaagtgggca cctccccgcg 960
atttcaagct gttacactct tccccccctt cggcctcttc tgtcgccttc taccgccctt 1020
ggacttctat ttcggtagaa gtgccacttg ttccttgccc aagacatcct cacgcgttat 1080
actgtactta acaagcaggg tctcgctcca ctccttgtta taacaccatc acatgcgctt 1140
cttcttttca aaaaaacggc ttgggattaa caaacttgtt gtaattagct atctaacatc 1200
tccttgaccg gctcaaaatc accggctata actactgacc cttcctctcc ccttccccct 1260
tcctggaccc cttggcactg gactctggaa catccgcctg gagcccgcca cctgcattcc 1320
caaggattga cccgcccctc ccgttggccc tcataacctt cgccattact cactataaac 1380
gccatgtctc gaaacgctcc caacgggtgt gttccaccct cccaagctac tgctccgcct 1440
tcgccagcca caagtctgcg actaacagtt ggggaaccgg tcagcgagcc ggccactgaa 1500
tccggggaga gagttctcca ggatgggttc tgggagcacg gtcgctttta tggttcttgg 1560
aagcctggga aatacctttt ccccatagac aaggtttgtc tctttgtagc gtcaataccc 1620
tccgcgcctg ttcgtacaac taacaacatc accaggagga gctcaatagg ttagatgtct 1680
ttcacaagta tttcctcgtt gcaagagacg agaaagtcac ttcaactccc ctgaggaaag 1740
atggacggcc gaaaatcatg gatctcggca caggcacggg catctgggcg tataatgttg 1800
tggaagagta agttattata gaggtagttt cacactacgc tgtgccagtt gctcacattt 1860
ttcaggtatg ccaaggatgc cgaaatcatg gccgtggatc tcaatcaaat tcaaccagct 1920
ctgtaagttg tgagctttca atcgcttgac cttttttttt tttttttttt tcaaacgcta 1980
atgcatttgc tgaatagcat tcctcgaggt gtaacaacca agcagtttga cattgaagag 2040

ccctcgtggg atccactgct tcgggactgc gaattgatcc atatgcgatt gctatacggc 2100
agcataagag atgacaagtg gccccatgtc taccgcaagg cctttgagtg cgtaactcgt 2160
gtaaccaaca acctgctccg tttctgacgt tcgtttacag gcacttggcc cctggcattg 2220
gctatatcga gcaactggag attgactgga tgccgcgatg ggagaatgag gatctcccca 2280
gacattcggc tcttcaagaa tgggctcagc tattccaacg tgccatgcat cgctaccacc 2340
gcagcgtcac ggtatcaggc gaggctacca gacgcagaat ggaagcggct ggctttacag 2400
atttctccga aacaacgatc cggtgctacg taaacccgtg gtctcccgat cgccatcagc 2460
gggagtgtgc ccgttggttc aacctcgcct tcagcctcgg ccttgaggcc atgagcatga 2520
tgccaatgat tgacaaactc ggcatgacca aggacgatat cgtcgacctc tgtagcagag 2580
ccaagaagga gatgtgcatt ctgcggtacc gcgcctattg cactctgtaa gccgcccgcc 2640
cccctgacaa acacaatttg ccggagccac aactaatcac gcttgcagac acatttggac 2700
agccagaaaa ccgaacgaag atgagtctca aactttcaaa gaaagagact ccgatacgca 2760
gccatctaga agagaggaat cctctgctta agacgccatg gtcgccaata gatcggatag 2820
agaagagaaa cagcatcctt gcgtccacat aatacaaacg gcgaccggca gggatgcgag 2880
agccagcgtt ctgcggcttg tcctgttttc gaagctacag aagcccaccg tgctatgtga 2940
gctaccgctt catatatgat cgtcgccgac gctgaacaat gtcatgcaac catcaccaag 3000
attctcatac aggtatagac gaagcctatt caagaacgag aagtatgcga ggtgtggata 3060
ctttggctct gtctcattcg aatgaagcac tgctagtggg ttcatggccg gatatatgat 3120
ctctcgtctt cgttctttac gctcttgacg atatgatgcc cgacaaaggc cagcccgttt 3180
ggttacaatg aaagatgacc ttgggcttag acctactcct cttctctccc tcttccttca 3240
tcctttctct tccccttgat cccgaggact tatcacatga cgacgaagaa cggggaaact 3300
ctgatggcta gaagcattgt agggacttag acgaaggtgg ggaacaggtg tacagaaaca 3360
tgtcggccag agtgttatgg gactcggccg ttgcaaacac gaagagatgg cgataagagc 3420
ctgaatgggg ttggcgttac aggggcattg tgtatggctt tactctctgc tgtcgattgg 3480
atagatcgtg tctttcgaac ttgaggatgt tgacatcact cgtgctggtt tttctgatta 3540
tgttgttggt taatcgcttc tgctagcagg ggcatctcgg caaggggtgg gcatgaccaa 3600
gagatgccgg aatcacccca ttacgaaaca ctacccaagc tgctaaaaca tccccatgtg 3660
gccagatgca aagggaacga aaaaaagaag aagagtaaag aaaaagcaaa caacactttg 3720
aagatatact attagggccc tttgtatgat acacttgaga ctgcctcctg catggtttca 3780
tctgggacgc tgatgcatgg ataccagcgc catgtaaccc gggacaaggt ccccttaggt 3840
ttgggtagtc taggtggtaa cctaggccag tagacagggg aagggtatgg gggcagaccg 3900
ggcaaatcat ttcagggacg gggcagcaaa ctacgagtga aagattgaga ggccgagagg 3960
aaacttgata cgggtggaaa gagtttgctt ctgttcaaag ggggatgttg ttggagaatg 4020
gaaagcgtga gtcttttggt gagaatgatg ttgtgatgtt g 4061

<210> 4

<211>  24
<212>  DNA
<213>  Artificial Sequence

<220>
<223>  Primer

<400>  4
taagcttcac tcgcttgtgt cttc                                              24


<210>  5
<211>  27
<212>  DNA
<213>  Artificial Sequence

<220>
<223>  Primer

<400>  5
tctcgagcgt ttatagtgag taatggc                                           27


<210>  6
<211>  26
<212>  DNA
<213>  Artificial Sequence

<220>
<223>  Primer

<400>  6
tctcgagcta ttgcactctg taagcc                                            26


<210>  7
<211>  26
<212>  DNA
<213>  Artificial Sequence

<220>
<223>  Primer

<400>  7
tgggccctgg gtagtgtttc gtaatg                                            26


<210>  8
<211>  26
<212>  DNA
<213>  Artificial Sequence

<220>
<223>  Primer

<400>  8
tctgcagacg agagatcata tatccg                                            26


<210>  9
<211>  25
<212>  DNA
<213>  Artificial Sequence

<220>
<223>  Primer

<400>  9
tactagtcta cctctttcaa ggagc                                             25

<210>  10
<211>  25
<212>  DNA
<213>  Artificial Sequence

<220>
<223>  Primer

<400>  10
tctgcagaaa gctaggagat cgtcc                                              25


<210>  11
<211>  25
<212>  DNA
<213>  Artificial Sequence

<220>
<223>  Primer

<400>  11
taagcttctc ttgcatcttt gtttg                                              25

## Description

The present invention relates to a transcription promoting protein methyltransferase of Trichoderma reesei.

It is known in the art that filamentous fungi such as Trichoderma reesei can be used to produce valuable compounds. Due to their glycosylation and secretion capacities, filamentous fungi are preferred hosts for producing secreting proteins.

Most of the industrial production of enzymes for plant biomass hydrolysis is performed with mutants of the fungus Trichoderma reesei (the anamorph of the tropical ascomycete Hypocrea jecorina). It is known that the genome contains a lower number of cellulase and hemicellulase genes than the genomes of other fungi. Although the number of said genes is relatively low Trichoderma reesei shows a relatively high expression of cellulases and hemicellulases which indicates that the expression of these enzymes is somehow up-regulated.

It is an object of the present invention to provide means to improve the expression of homologous and heterologous proteins, in particular enzymes, in fungi, in particular in Trichoderma reesei. This means should be able to up- as well as down-regulate protein expression in fungi. Furthermore this means may also be used in recombinant protein expression.

Therefore the present invention relates to an isolated polypeptide with protein methyltransferase activity having at least 80% identity with amino acid sequence SEQ ID No. 1.

It surprisingly turned out that the polypeptide of the present invention, which may be encoded by nucleotide sequence SEQ ID No. 2 from Trichoderma reesei, is able to regulate the expression rate of a specific group of proteins which are all related to biomass degradation (carbohydrate-active enzymes, CAZymes). It could be shown herein that if the concentration of the polypeptide of the present invention is reduced within the cell the amount of the aforementioned proteins is also reduced. On the other hand an increase of the concentration of the polypeptide of the present invention within the cell leads to an increased production of CAZymes. This data clearly demonstrate that the regulation of the expression of the protein methyltransferase having at least 80% identity with amino acid sequence SEQ ID No. 1 allows the regulation of the expression rate of specific proteins within a cell.

The above described effect is a result of the fact that biomass degrading proteins are usually clustered within the genome of fungi like the enzymes involved in the biosynthesis of secondary metabolites. The latter are known to occur in clusters, frequently near the telomere end of the chromosomes. In another fungal genus, the Aspergilli, such clusters of secondary metabolite genes have been demonstrated to be epigenetically regulated at an upper hierarchic level by the protein methyltransferase LaeA, by reversing the repressing heterochromatin structure resulting from methylation of K9 on histone 3A and binding of the heterochromatin protein HepA to histone 3A. Because of the clustered co-occurrence of cellulase and secondary metabolite synthesis genes in the Trichderma reesei genome, cellulase formation is regulated by an LaeA orthologue which exhibits at least 80% identity with amino acid sequence SEQ ID No. 1.

As indicated in Table 1 of the examples section the regulation of the expression of the polypeptide having amino acid sequence SEQ ID No. 1 results in the regulation of the protein expression of the following proteins (classification in accordance with Henrissat B and Bairoch A, Biochem J. 316(1996): 695-696; www.cazy.org/Glycoside-Hydrolases.html): GH 5 endo-β-1,4-glucanase CeI5A, GH5 endo-β-1,4-glucanase CEL5B, GH6 Cellobiohydrolase 2 CEL6A, GH7 endo-β,4-glucanase EGL1, GH7 cellobiohydrolase 1 CEL7A, GH12 endo-β-1,4-glucanase 12a, GH45 endo-β-1,4-glucanase EG5, GH61 endo-β-1,4-glucanase CEL61A, GH61endo-β-1,4-glucanase CEL61B, GH1 β-glucosidase CEL1B, GH1 β-glucosidase CEL1A, GH3 β-glucosidase of uncertain specificity, GH3 β-glucosidase CEL3D, GH3 β-glucosidase CEL3C, CIP2, CIP1, CBM13 protein, swollenin, swollenin-like, 84 % ID to 123992, GH10 xylanase XYN3, GH11xylanase XYN1, GH11 xylanase XYN2, GH30 xylanase XYN4, GH3 β-xylosidase BXL1, GH43 β-xylosidase/α-arabinofuranosidase, GH74 xyloglucananase CEL74a, hemicellulose side chain cleaving enzymes, CE5 acetyl xylan esterase AXE1, GH67 α-glucuronidase AGU1, GH62, α-L-arabinofuranosidase ABF2, GH54, L-α-arabinofuranosidase ABF1, GH95 α-fucosidase, GH95 α-fucosidase, GH92 α-1,2-mannosidase, GH92 α-1,2-mannosidase, GH47 α-1,2-mannosidase, GH2 β-mannosidase, GH27 α-galactosidase AGL1, GH27 α-galactosidase AGL3 and GH28 polygalacturonase.

The findings of the present invention can be used to provide host cells, in particular genetically modified fungi like Trichoderma reesei, which show an increased or reduced CAZyme expression activity. For instance, it is possible to provide Trichoderma reesei cells which do not express biomass degrading enzymes (by inactivating (e.g. gene deletion or disruption) of the gene encoding for the protein methyltransferase of the present invention). However, it is also possible to provide cells which show a high activity of said enzymes. The latter effect can be achieved by increasing the expression rate of the protein methyltransferase according to the present invention. This can be achieved by introducing some more copies of a nucleic acid molecule harboring a nucleic acid stretch encoding for the protein methyltransferase of the present invention. Alternatively the promoter region of the native gene may be modified to comprise homologous or heterologous promoter which are much stronger than the native promoter. In order to produce heterologous proteins in a fungus like Trichoderma reesei under the control of the protein methyltransferase of the present invention one of the above identified enzymes, which are regulated by said protein methyltransferase, may be exchanged by genetic manipulation by a heterologous nucleic acid molecule encoding for a product of interest. Since the genome sequence of Trichoderma reesei, for instance, is known in the art such manipulations can be easily performed.

Methods for the production of such genetically modified fungi, in particular Trichoderma reesei, are well known in the art. For instance, in WO 2006/060126 methods for transforming and cultivating Trichoderma reesei cells are disclosed.

It is emphasized that also a functional fragment of the polypeptide showing protein methyltransferase activity and having at least 80% identity with amino acid sequence SEQ ID No. 1 is subject of the present invention. "Functional fragments" of the protein methyltransferase of the present invention refer to possible fragments which still retain the activity of the full length polypeptide.

According to the present invention the polypeptide showing transcription promoting activity may be at least 80%, preferably at least 90%, more preferably at least 95%, even more preferably at least 98%, in particular 100%, identical with amino acid sequence SEQ ID No. 1.

SEQ ID No. 1:

MSRNAPNGCVPPSQATAPPSPATSLRLTVGEPVSEPATESGERVLQDGFWEHGRFYGSWKPGKY
LFPIDKEELNRLDVFHKYFLVARDEKVTSTPLRKDGRPKIMDLGTGTGIWAYNVVEEYAKDAEI
MAVDLNQIQPALIPRGVTTKQFDIEEPSWDPLLRDCELIHMRLLYGSIRDDKWPHVYRKAFEHL
APGIGYIEQLEIDWMPRWENEDLPRHSALQEWAQLFQRAMHRYHRSVTVSGEATRRRMEAAGFT
DFSETTIRCYVNPWSPDRHQRECARWFNLAFSLGLEAMSMMPMIDKLGMTKDDIVDLCSRAKKE
MCILRYRAYCTL

A further aspect of the present invention relates to an isolated nucleic acid molecule encoding an isolated polypeptide having at least 80% identity with amino acid sequence SEQ ID No. 1.

The nucleic acid molecule according to the present invention exhibits preferably at least 80% identity with nucleic acid sequence SEQ ID No. 2.

SEQ ID No. 2:

ATGTCTCGAAACGCTCCCAACGGGTGTGTTCCACCCTCCCAAGCTACTGCTCCGCCTTCGCCAG
CCACAAGTCTGCGACTAACAGTTGGGGAACCGGTCAGCGAGCCGGCCACTGAATCCGGGGAGAG
AGTTCTCCAGGATGGGTTCTGGGAGCACGGTCGCTTTTATGGTTCTTGGAAGCCTGGGAAATAC
CTTTTCCCCATAGACAAGGAGGAGCTCAATAGGTTAGATGTCTTTCACAAGTATTTCCTCGTTG
CAAGAGACGAGAAAGTCACTTCAACTCCCCTGAGGAAAGATGGACGGCCGAAAATCATGGATCT
CGGCACAGGCACGGGCATCTGGGCGTATAATGTTGTGGAAGAGTATGCCAAGGATGCCGAAATC
ATGGCCGTGGATCTCAATCAAATTCAACCAGCTCTGCACTTGGCCCCTGGCATTGGCTATATCG
AGCAACTGGAGATTGACTGGATGCCGCGATGGGAGAATGAGGATCTCCCCAGACATTCGGCTCT
TCAAGAATGGGCTCAGCTATTCCAACGTGCCATGCATCGCTACCACCGCAGCGTCACGGTATCA
GGCGAGGCTACCAGACGCAGAATGGAAGCGGCTGGCTTTACAGATTTCTCCGAAACAACGATCC
GGTGCTACGTAAACCCGTGGTCTCCCGATCGCCATCAGCGGGAGTGTGCCCGTTGGTTCAACCT
CGCCTTCAGCCTCGGCCTTGAGGCCATGAGCATGATGCCAATGATTGACAAACTCGGCATGACC
AAGGACGATATC

Nucleic acid sequence SEQ ID No. 2 is directly derived from Trichoderma reesei. However, according to the present invention this sequence may of course vary provided that the encoded protein still exhibits the transcription promoting protein methyltransferase of the polypeptide disclosed herein.

According to the present invention the nucleic acid molecule exhibits at least 80%, preferably at least 90%, more preferably at least 95%, even more preferably at least 98%, in particular 100%, identity with nucleic acid sequence SEQ ID No. 2.

A further aspect of the present invention relates to a vector comprising an isolated nucleic acid molecule according to the present invention.

In order to transfer a nucleic acid molecule encoding the polypeptide of the present invention to a host cell the nucleic acid molecule of the present invention is provided in a vector. The vector may be an expression vector capable to express the polypeptide of the present invention. However, the vector of the present invention may also be a recombination vector which allows to transfer a nucleic acid molecule encoding the polypeptide of the present invention into the genome of a host cell. The vector of the present invention may comprise further elements such as additional coding sequences within the same transcription unit, controlling elements such as promoters, ribosome binding sites, transcription terminators, polyadenylation sites, additional transcription units under control of the same or different promoters, sequences that permit cloning, expression, homologous recombination, and transformation of a host cell.

The vector of the present invention preferably comprises further at least one promoter operably linked to said nucleic acid molecule.

In order to control the transcription of the nucleic acid molecule of the present invention within the cell at least one promoter is provided within the vector. "Operably linked" refers to a juxtaposition wherein the components so described are in a relationship permitting them to function in their intended manner. A promoter "operably linked" to a coding sequence is present in the cell in such a way that expression of the coding sequence can be directly influenced by the promoter.

According to a preferred embodiment of the present invention the at least one promoter is selected from the group consisting of *tef1* promoter (transcription elongation factor 1), *gpg1* promoter, *pki1* promoter, enol promoter and *pgk1* promoter.

Of course it is also possible to use any other promoter which can be used to regulate gene expression in host organisms such as Trichoderma reesei. Particularly preferred promoters are those regulating the protein expression of the following proteins in Trichoderma reesei: GH5 glycoside hydrolase (protein ID 81087), Bradorhizobium bleomycin resistance (protein ID 103009), unknown hypothetical protein (protein ID 106270), HHE domain protein, conserved (protein ID 70608), hypothetical conserved protein (protein ID 109925), PTH11-type GPCRs (protein ID 109146), MSF permease (protein ID 78585), glutathione-S-transferase (protein ID 112022), Catalase (protein ID 58472), mannose-6-phosphate isomerase (protein ID 60445), unknown protein (protein ID 105287), translation initiation regulator Gnc20 (protein ID 22839), imidazole proprionase-related amidohydrolase (protein ID 110757), MSF transporter (protein ID 3405), short chain dehydrogenase/reductase (protein ID 106164), MSF permease (protein ID 79202), unknown protein (protein ID 60370), hypothetical secreted protein (protein ID 122889), Flavonol reductase/cinnamoyl-CoA reductase (protein ID 111716), Zinc-binding oxidoreductase (protein ID 23292), hypothetical protein (protein ID 124198), unknown protein (protein ID 109523), Kynurenine aminotransferase, glutamine transaminase K (protein ID 122820), GPR1/FUN34/yaaH-like protein (protein ID 60810), hypothetical protein (protein ID 54352), Predicted Zn-dependent hydrolase (beta-lactamase superfamily) (protein ID 70197), sulfite transporter ssu1 (protein ID 2076), unknown protein (protein ID 4851), unknown secreted protein (protein ID 110830), unknown esterase/lipase (protein ID 70491) and MSF permease (protein ID 105260). These genes are regularly upregulated under cellulose inducing conditions (e.g. cultivation of Trichoderma reesei on lactose).

The vector according to the present invention, which can be transformed into a Trichoderma reesei cell comprises in the 5' region of the nucleic acid molecule exhibiting at least 80% identity with nucleic acid sequence SEQ ID No. 2 or of the nucleic acid molecule encoding for a polypeptide having at least 80% identity with amino acid sequence SEQ ID No. 1 a promoter which allows to control the expression rate of the polypeptide of the present invention within a cell. Preferred promoters are selected from the group of promoters selected from the group consisting of cbh1, cbh2, xyn1, xyn2, xyn3, egl1, gna3, env1, cDNA1, bxl1, pki1, gpdA, gpd1 or hex1 promoters. Preferred promoters are also promoters disclosed, for instance, in Nakari-Setäla et al. (Appl. Env. Microbiol. 61 (1995), 3650-3655; cDNA1 promotor), Rahman et al. (Biosci. Biotechnol. Biochem. 73 (2009), 1083-1089; egl3 and xyn3 promoter), WO 98/23764 A1 (short cbh1 promoter), EP 0 952 223 A1 (cbh1-promoter of Trichoderma viridae), US 7,393,664, US 7,517,685 (hex1 promoter) and Mach et al. (Curr. Genetics 25 (1994): 567-570; pki1). In a preferred embodiment of the present invention the promoters may be of heterologous or homologous origin.

Suitable promoters can also be provided by using the method disclosed in US 5,989,870.

Another aspect of the present invention relates to a recombinant host cell comprising a nucleic acid molecule or a vector according to the present invention.

In order to increase the amount of the polypeptide of the present invention within the host cell more than one copy of the nucleic acid molecule or vector according to the present invention are preferably provided in said cells.

According to a preferred embodiment of the present invention the host cell is a fungus, preferably a fungus of the class of Sordariomycetes, more preferably a fungus of the family of Hypocreaceae, even more preferably a fungus of the genus of Trichoderma, in particular Trichoderma reesei.

A further aspect of the present invention relates to a genetically modified Trichoderma reesei cell overexpressing a polypeptide with transcription promoting protein methyltransferase having at least 80% identity with amino acid sequence SEQ ID No. 1 compared to a wild-type Trichoderma reesei cell.

The Trichoderma reesei cell according to the present invention comprises preferably a vector according as defined above.

According to a preferred embodiment of the present invention said cell comprises at least one mutation within the 5'-region of the genome location comprising nucleic acid sequence SEQ ID No. 2 or a variant thereof having at least 80% identity to SEQ ID No. 2.

The Trichoderma reesei genome location comprising the 5' and 3' region of nucleic acid SEQ ID No. 2 comprises the following nucleotide sequence (SEQ ID No. 3; italic: intron, underlined: coding region) :

CCTTTTACCAACTTGGCAGCCCTTGCCTCTTCGTTGCTGGCTAGTAGGGGAGGCAGGCCATTGATCCCGG
GCTCGCGTCAATCCACCAAGCCCCAAAGAGCCCTAGAAGCTCGCGACACTTGTCATTGAACCAACACGAC
TCTCAACCGCCGTCTGTCGATTCTCACTTCGGCATTCGTCGACCTCCTCCCTCCAGCCGCTGGTCCACTC
CGGACCCGAGCTTCGCGCCAGTCTTAAAGGGCTAGCCGTCCTCGCCCCCCCTTCTCCAGTCCGCCGAACG
ACAGTCTCAACCTCAACCCTGGGAGTCGTGATAATTTTCTATACCGCCCTTCTCCGCTCTCTCAGCCGTC

ACTCGCTTGTGTCTTCCAACCGGAGCTGTCCACGCTGCCGCCTGGGACGATTACGCCCTTGCCTGTCGCT
GCATTCGTGCTGCAAATTGGTTGCTCCGGTGGCTCAGGTGACCCTGGTCGAGGACTTCTACCTCTTTCAA
GGAGCCTCGTGAGAGTTGATACAGCAGCTGCCCTCGACAGGTCACACAAACACATGGATCGTCGCTTAT
TCTGACCTACGGCAGCTGCGCGGCAACCCACCCGTACCACAGGCATCGGTATCTGTGTCACCTCCTTGAC
ATCTCGGCCAGTTGAATTTCTGATGTAAGTTGCCATCTGCCTTGTTGTTGTTCGTCTGGCTGCCCTACAC
CATCTTGCCCGCCTGCTCGGCTCCCATCTACTCCTTGATTTATCTTTCCATCTTGTCTTACTGCCATGAT
GCGCGTTGAAAGGCGCCATGCATGCCATCGTGTCCGGCCAGGATATGAATGCTGTGCTCAGTCAGCCGTC
TTCCTTTTTGGAAGCCAATCAGCAAGGTTGAACTGTCTTTCTCTTTCCTTGCCATCATCAACCCTTGCGG
CAGATGTTTATGTCTCTCGCCCCTTGCCTTGAAGTGGCACCTCCCCGCGATTTCAAGCTGTTACACTCT
TCCCCCCCTTCGGCCTCTTCTGTCGCCTTCTACCGCCCTTGGACTTCTATTTCGGTAGAAGTGCCACTTG
TTCCTTGCCCAAGACATCCTCACGCGTTATACTGTACTTAACAAGCAGGGTCTCGCTCCACTCCTTGTTA
TAACACCATCACATGCGCTTCTTCTTTTCAAAAAAACGGCTTGGGATTAACAAACTTGTTGTAATTAGCT
ATCTAACATCTCCTTGACCGGCTCAAAATCACCGGCTATAACTACTGACCCTTCCTCTCCCCTTCCCCCT
TCCTGGACCCCTTGGCACTGGACTCTGGAACATCCGCCTGGAGCCCGCCACCTGCATTCCCAAGGATTGA
CCCGCCCCTCCCGTTGGCCCTCATAACCTTCGCCATTACTCACTATAAACGCCATGTCTCGAAACGCTCC
CAACGGGTGTGTTCCACCCTCCCAAGCTACTGCTCCGCCTTCGCCAGCCACAAGTCTGCGACTAACAGTT
GGGGAACCGGTCAGCGAGCCGGCCACTGAATCCGGGGAGAGAGTTCTCCAGGATGGGTTCTGGGAGCACG
GTCGCTTTTATGGTTCTTGGAAGCCTGGGAAATACCTTTTCCCCATAGACAAGGTTTGTCTCTTTGTAGC
GTCAATACCCTCCGCGCCTGTTCGTACAACTAACAACATCACCAGGAGGAGCTCAATAGGTTAGATGTCT
TTCACAAGTATTTCCTCGTTGCAAGAGACGAGAAAGTCACTTCAACTCCCCTGAGGAAAGATGGACGGCC
GAAAATCATGGATCTCGGCACAGGCACGGGCATCTGGGCGTATAATGTTGTGGAAGAGTAAGTTATTATA
GAGGTAGTTTCACACTACGCTGTGCCAGTTGCTCACATTTTTCAGGTATGCCAAGGATGCCGAAATCATG
GCCGTGGATCTCAATCAAATTCAACCAGCTCTGTAAGTTGTGAGCTTTCAATCGCTTGACCTTTTTTTTT
TTTTTTTTTTCAAACGCTAATGCATTTGCTGAATAGCATTCCTCGAGGTGTAACAACCAAGCAGTTTGA
CATTGAAGAGCCCTCGTGGGATCCACTGCTTCGGGACTGCGAATTGATCCATATGCGATTGCTATACGGC
AGCATAAGAGATGACAAGTGGCCCCATGTCTACCGCAAGGCCTTTGAGTGCGTAACTCGTGTAACCAACA
ACCTGCTCCGTTTCTGACGTTCGTTTACAGGCACTTGGCCCCTGGCATTGGCTATATCGAGCAACTGGAG
ATTGACTGGATGCCGCGATGGGAGAATGAGGATCTCCCCAGACATTCGGCTCTTCAAGAATGGGCTCAGC
TATTCCAACGTGCCATGCATCGCTACCACCGCAGCGTCACGGTATCAGGCGAGGCTACCAGACGCAGAAT
GGAAGCGGCTGGCTTTACAGATTTCTCCGAAACAACGATCCGGTGCTACGTAAACCCGTGGTCTCCCGAT
CGCCATCAGCGGGAGTGTGCCCGTTGGTTCAACCTCGCCTTCAGCCTCGGCCTTGAGGCCATGAGCATGA
TGCCAATGATTGACAAACTCGGCATGACCAAGGACGATATCGTCGACCTCTGTAGCAGAGCCAAGAAGGA
GATGTGCATTCTGCGGTACCGCGCCTATTGCACTCTGTAAGCCGCCCGCCCCCCTGACAAACACAATTTG
CCGGAGCCACAACTAATCACGCTTGCAGACACATTTGGACAGCCAGAAAACCGAACGAAGATGAGTCTCA
AACTTTCAAAGAAAGAGACTCCGATACGCAGCCATCTAGAAGAGAGGAATCCTCTGCTTAAGACGCCATG
GTCGCCAATAGATCGGATAGAGAAGAGAAACAGCATCCTTGCGTCCACATAATACAAACGGCGACCGGCA
GGGATGCGAGAGCCAGCGTTCTGCGGCTTGTCCTGTTTTCGAAGCTACAGAAGCCCACCGTGCTATGTGA
GCTACCGCTTCATATATGATCGTCGCCGACGCTGAACAATGTCATGCAACCATCACCAAGATTCTCATAC
AGGTATAGACGAAGCCTATTCAAGAACGAGAAGTATGCGAGGTGTGGATACTTTGGCTCTGTCTCATTCG
AATGAAGCACTGCTAGTGGGTTCATGGCCGGATATATGATCTCTCGTCTTCGTTCTTTACGCTCTTGACG

ATATGATGCCCGACAAAGGCCAGCCCGTTTGGTTACAATGAAAGATGACCTTGGGCTTAGACCTACTCCT
CTTCTCTCCCTCTTCCTTCATCCTTTCTCTTCCCCTTGATCCCGAGGACTTATCACATGACGACGAAGAA
CGGGGAAACTCTGATGGCTAGAAGCATTGTAGGGACTTAGACGAAGGTGGGGAACAGGTGTACAGAAACA
TGTCGGCCAGAGTGTTATGGGACTCGGCCGTTGCAAACACGAAGAGATGGCGATAAGAGCCTGAATGGGG
TTGGCGTTACAGGGGCATTGTGTATGGCTTTACTCTCTGCTGTCGATTGGATAGATCGTGTCTTTCGAAC
TTGAGGATGTTGACATCACTCGTGCTGGTTTTTCTGATTATGTTGTTGGTTAATCGCTTCTGCTAGCAGG
GGCATCTCGGCAAGGGGTGGGCATGACCAAGAGATGCCGGAATCACCCCATTACGAAACACTACCCAAGC
TGCTAAAACATCCCCATGTGGCCAGATGCAAAGGGAACGAAAAAAAGAAGAAGAGTAAAGAAAAAGCAAA
CAACACTTTGAAGATATACTATTAGGGCCCTTTGTATGATACACTTGAGACTGCCTCCTGCATGGTTTCA
TCTGGGACGCTGATGCATGGATACCAGCGCCATGTAACCCGGGACAAGGTCCCCTTAGGTTTGGGTAGTC
TAGGTGGTAACCTAGGCCAGTAGACAGGGGAAGGGTATGGGGGCAGACCGGGCAAATCATTTCAGGGACG
GGGCAGCAAACTACGAGTGAAAGATTGAGAGGCCGAGAGGAAACTTGATACGGGTGGAAAGAGTTTGCTT
CTGTTCAAAGGGGGATGTTGTTGGAGAATGGAAAGCGTGAGTCTTTTGGTGAGAATGATGTTGTGATGTT
G

The 5' region of SEQ ID No. 2 as evidenced above (SEQ ID No. 3) may be mutated by incorporating additional or alternative regulatory sequences. These regulatory sequences include promoters, such as those listed above in connection with the vectors of the present invention.

According to a preferred embodiment of the present invention the at least one mutation is a deletion, an insertion or a point mutation.

According to a further preferred embodiment of the present invention said Trichoderma reesei cell comprises within the 5'-region of the genome location comprising nucleic acid sequence SEQ ID No. 2 a promoter, a transcription factor binding site or a functional fragment thereof is inserted resulting in an increased expression of the polypeptide encoded by the nucleic acid sequence SEQ ID No. 2 or a variant thereof having at least 80% identity to SEQ ID No. 2 compared to the wild-type Trichoderma reesei.

The Trichoderma reesei cell of the present invention preferably comprises further a recombinant nucleic acid coding region operatively linked to a Trichoderma reesei promoter sequence and/or a vector comprising a nucleic acid coding region operatively linked to a Trichoderma reesei promoter sequence, wherein the nucleic acid coding region is under the transcriptional control of said promoter sequence.

The nucleic acid coding region encodes preferably for a peptide, a polypeptide, a protein or a functional DNA or RNA.

According to a preferred embodiment of the present invention a nucleic acid molecule encoding for a protein, polypeptide, peptide or functional DNA of interest may be introduced into the genome of Trichoderma reesei by gene replacement at the genome location comprising one or more of the genes encoding the proteins listed in table 1 of the example section. Methods for performing a gene replacement are well known in the art (see e.g. Guangtao Z et al., J. Biotechnol. 139 (2009): 146-151). The genomic sequence of Trichoderma reesei is known in the art (Martinez D et al., Nat. Biotechnol. 26 (2008): 553-560), therefore the gene replacement can easily be performed using the methods known in the art. The following genes are examples that are abundantly expressed on lactose, and present in LAE1-regulated genomic clusters: alcohol oxidase AOX1 (Trire2: 80659), a hexose transporter (Trire2: 105260), the lactate/pyruvate transporter (Trire2: 121441), a major facilitator superfamily protein (Trire2:70972), and GPR1 (Trire2:60810).

Alternatively, if the purpose is heterologous overexpression of proteins, the following cellulase genes could be replaced: Ce16A (Trire2:72567), Ce17A (Trire2:123989), Ce161B (Trire2: 120961), and Cel5A (Trire2:120312)

Yet another aspect of the present invention relates to a method for the recombinant production of a peptide, a polypeptide, a protein or a functional DNA or RNA comprising the step of cultivating a genetically modified Trichoderma reesei cell according to the present invention.

As described above and shown in the examples the expression of specific proteins in Trichoderma reesei is controlled by the protein methyltransferase of the present invention. This control mechanism allows to generate recombinant Trichoderma reesei cells which harbor heterologous nucleic acid molecules within the genomic loci of the nucleic acid molecules naturally regulated by the polypeptide of the present invention within the cell. Of course, it is also possible to exploit this effect to overexpress biomass degrading enzymes naturally occurring in Trichoderma reesei cells and to isolate them. To achieve this object genetically modified Trichoderma reesei cells as described above have to be used which overexpress the polypeptide of the present invention in comparison to wild-type Trichoderma reesei cells.

The present invention is further illustrated by the following figures and examples, however, without being restricted thereto.

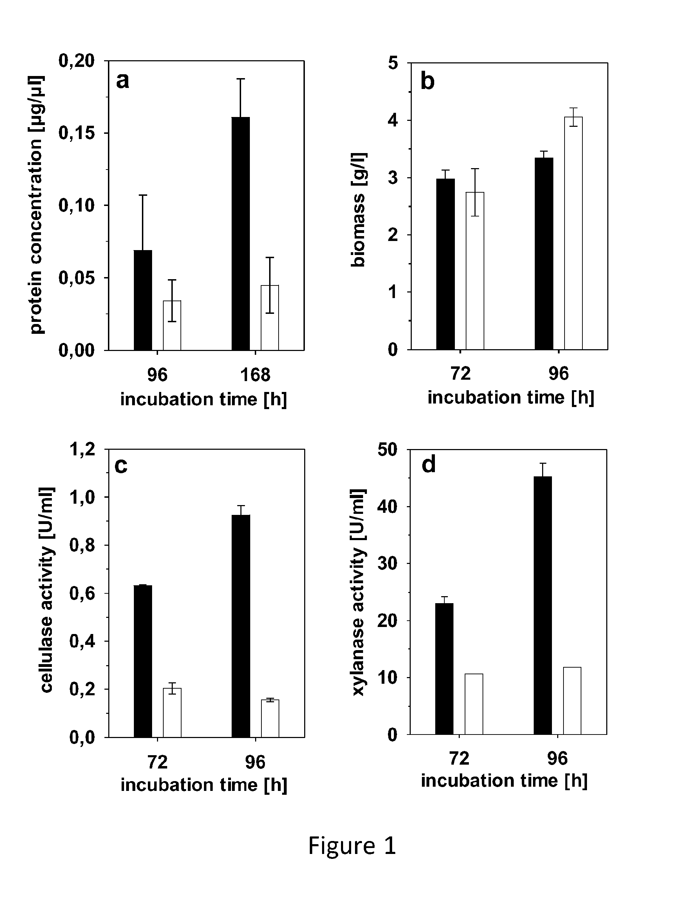

Fig. 1 shows the effect of loss-of-function of lae1 on biomass formation and cellulase/hemicellulase enzyme formation by *T. reesei.* Growth of *T. reesei* QM 9414 and the corresponding Δlae1 strain on 1 % (w/v) cellulose (a) and 1 % (w/v) glycerol (b). Biomass on cellulose is quantified as the fungal protein that can be extracted from the cellulose-fungus debris by 0.1 M NaOH (1 h, 30°C) and refered to 1 L of culture, whereas that on glycerol is given by the biomass dry weight per L. Cellulase (c) and hemicellulase (d) formation by *T. reesei* QM 9414 and the corresponding Δlae1 strain on 1 % (w/v) lactose and 1 % (w/v) xylan, respectively. Experiments are means of 3-5 biological replicas.

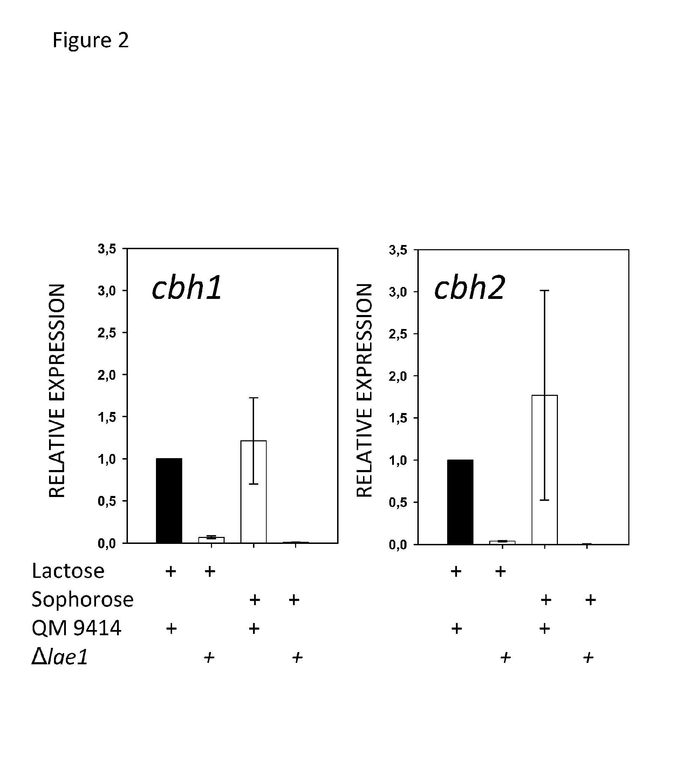

Fig. 2 shows the expression of the two cellulase genes cbh1, encoding CEL7A (a) and cbh2, encoding CEL6A (b) in *T. reesei* QM 9414 and the Δlae1 mutant during growth or incubation, respectively, on glycerol, lactose and sophorose. Expression in QM 9414 is given with full bars and set to 1.0 for every condition. The respective expression levels in relation to the wild-type are shown with open bars. Data are means of triplicate determinations from two biological replica.

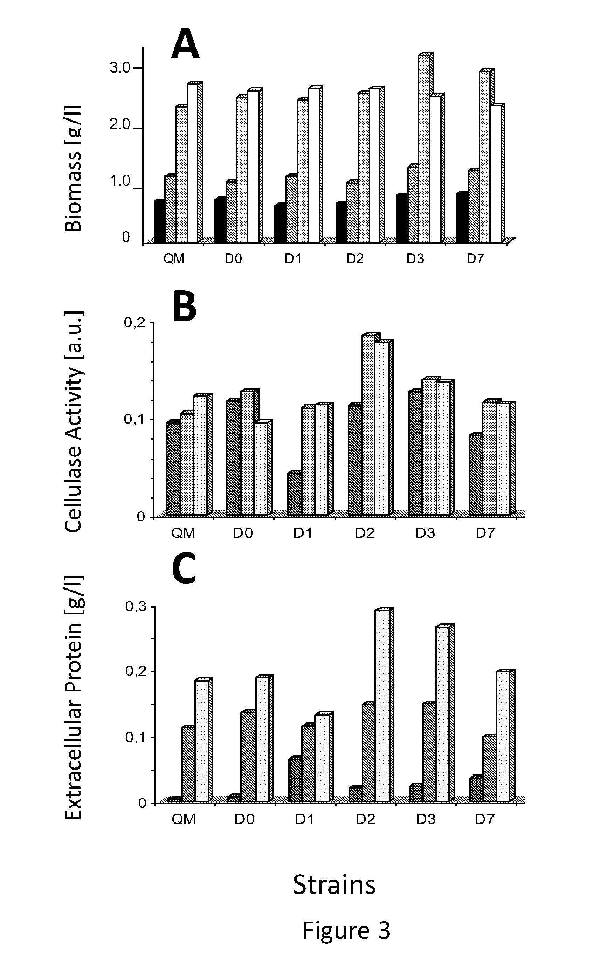

Fig. 3 shows the biomass formation (A), cellulase production (B) and extracellular protein (C) during growth of *T. reesei* QM 9414 (QM) and several mutant strains bearing an additional copy of the lae1 gene (DO, D1, D2, D3, D7) on lactose. The three bars represent (from left to right) values for 48, 72 and 96 hrs of cultivation.

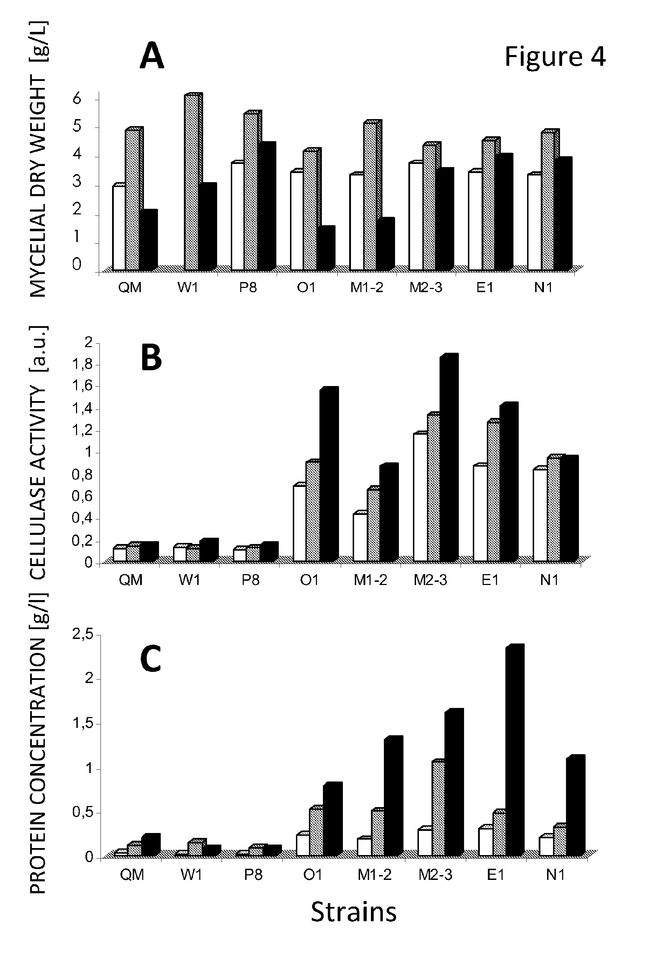

Fig. 4 shows the biomass formation (A), cellulase production (B) and extracellular protein (C) during growth of T. reesei QM 9414 (QM) and several mutant strains bearing an additional copy of the tef1:lae1 gene construct(W1, P8, 01, M1-2, M2-3, E1, N1) on lactose. The three bars represent (from left to right) values for 48, 72 and 96 hrs of cultivation.

### EXAMPLE:

### Materials and Methods

### Strains

*T. reesei* QM9414 (ATCC 26921), an early cellulase producing mutant and *H. jecorina* KU70, a derivative of the QM 9414 uridine auxotrophic pyr4 negative strain TU-6 (ATCC MYA-256), and which bears a deletion in the ku70 gene and is thus deficient in non-homologous end joining, were used in this example. *Escherichia coli* JM109 (Promega, USA) was used for plasmid construction and amplification.

### Construction of a Δlae1 strain of T. reesei

To delete the lae1 gene of *T. reesei,* a 1.2 kb lae1 coding region was replaced by the *T. reesei* pyr4 (orotidine 5'-phosphate decarboxylase-encoding) gene. This was performed by amplifying around 1 kb of the up- and downstream non-coding region of lae1 from genomic DNA of T. reesei QM9414 using the primer pairs given in the following table:

**Table A: Oligonucleotide primers used for construction of vectors for lae1 deletion and amplification**

| **Name** | | ***Sequence** |
|---|---|---|
| | *lae1 gene deletion* | |
| 5Tr1ae1Hind | | TAAGCTTCACTCGCTTGTGTCTTC |
| 5Trlae1Xho | | TCTCGAGCGTTTATAGTGAGTAATGGC |
| 3Trlae1Xho | | TCTCGAGCTATTGCACTCTGTAAGCC |
| 3Trlae1Apa | | TGGGCCCTGGGTAGTGTTTCGTAATG |
| | *lae1 gene amplification* | |
| laelPstI | | TCTGCAGACGAGAGATCATATATCCG |
| lae1SpeI | | TACTAGTCTACCTCTTTCAAGGAGC |
| ptrAPstI | | TCTGCAGAAAGCTAGGAGATCGTCC |
| ptrAHindIII | | TAAGCTTCTCTTGCATCTTTGTTTG |

| | | |
|---|---|---|
| *Respective restriction sites are underlined | | |

Resulting PCR fragments were ligated by T/A cloning into pGEM-T Easy (Promega, USA). The upstream non-coding region was excised by digestion with XhoI/HindIII and the downstream region by XhoI/ApaI from the pGEM-T Easy backbone, and then both fragments were ligated into a ApaI/HindIII restricted vector pBluescript SK(+) (Stratagene, USA). The resulting plasmid was cleaved with XhoI, dephosphorylated and the 2.7 kb SalI fragment of T. *reesei* pyr4 inserted resulting in pΔlae1.

### lae1 gene amplification in T. reesei

To introduce a second copy of lae1 into the genome of *T*. *reesei* QM 9414, 900 bp of the upstream and 500 bp of the downstream non-coding region of lae1 were amplified from genomic DNA of *T. reesei* QM9414 using the primer pairs given in Table A. As a selection marker, 2 kb of the A. oryzae ptrA (pyrithiamine resistance conferring) gene was amplified from plasmid pME2892 (Kubodera et al. 2000) using the primer pair given in Table A. PCR fragments were cloned into pGEM-T Easy. lae1 was then excised with SpeI/PstI and ptrA by PstI/HindIII, respectively. lae1 was subsequently ligated into pBluescript SK(+), previously cut with SpeI/PstI, followed by the cloning of ptrA into the resulting plasmid plae1ptrA.

### mRNA extraction and Real Time PCR

DNase treated (DNase I, RNase free; Fermentas) RNA (5µg) was reverse transcribed with the RevertAid™ First Strand cDNA Kit (Fermentas) according to the manufacturer's protocol with a combination of the provided oligo-dT and random hexamer primers. All real-time RT-PCR experiments were performed on a Bio-Rad (USA) iCycler IQ. For the reaction the IQ SYBR Green Supermix (Bio-Rad, USA) was prepared for 25 µl assays with standard MgCl₂ concentration (3 mM) and a final primer concentration of 100 nM each. All assays were carried out in 96-well plates which were covered with optical tape. The amplification protocol consisted of an initial denaturation step (3 min at 95°C) followed by 40 cycles of denaturation (15 sec at 95°C), annealing (20 sec at 57°C) and elongation (10 sec at 72°C). Determination of the PCR efficiency was performed using triplicate reactions from a dilution series of cDNA (1.00E-00, 1.00E-01, 1.00E-02 and 1.00E-03). Amplification efficiency was then calculated from the given slopes in the IQ5 Optical system Software v2.0. Expression ratios were calculated using REST© Software (Pfaffl M.W. et al. Nucleic Acid Research 30(2009): e36)). All samples were analyzed in two independent experiments with three replicates in each run.

### Transcriptome analysis of lae1 loss-of function

Mycelia from both stages were freeze-dried and ground in liquid nitrogen using a mortar and pestle. For each of the two experimental conditions, five independent replicates of mycelium were mixed. Total RNAs were extracted using TRIzol® reagent (In-vitrogen Life Technologies, USA), according to the manufacturer's instructions, and then purified using the RNeasy MinE-lute Cleanup Kit (Qiagen, Germany). The RNA quality and quantity were determined using a Nanodrop spectrophotometer. High quality purified RNAs were submitted to Roche-NimbleGene (40 µg per 3-microarray set) where cDNAs were synthesized, amplified and la-beled and then used for subsequent hybridization.

A *T. reesei* high density oligonucleotide (HDO) microarray (Roche-NimbleGen, Inc., USA) was constructed, using 60-mer probes representing the 9.130 genes of *T. reesei.* Microarray scanning, data acquisition and identification of probe sets showing a significant difference (p=0.05) in expression level between the two culture conditions considered were performed by Roche-NimbleGen. Transcripts showing significantly up-regulated expression (2-fold and 5-fold changes) were annotated using the eukaryotic orthologous groups (KOG) classification. The microarray data and the related protocols are available at the GEO web site (www.ncbi.nlm.nih.gov/geo/) under accession number: GSE20516.

### Results

To identify lae1, 92 S-methionyl-adenosine-dependent methyltransferases present in the *T. reesei* genome database (http://genome.jgi-psf.org/Trire2/Trire2.home.html) were screened. When any of the functionally verified Aspergillus LaeA proteins was used as a query in BLASTP, several hits with negative probabilities of <e-30 were obtained, but using these as a query in BLASTP of the respective Aspergillus genome databases (http://www.broadinstitute.org/annotation/genome/aspergillus_gro up/MultiHome.html) always resulted in several hits of similar negative probability and thus identified none of them clearly as a LaeA orthologue. Since this approach was therefore prone to lead to false positives, an iterative phylogenetic strategy for its identification was used: briefly, BLASTP was used to detect LaeA orthologues in species more closer related to the Aspergilli (such as Coccidioides immitis) and then used the identified protein to look for LaeA orthologues in Dothidiomycetes, and used the latter one to the Sordariomycetes and finally the Hypocreaceae. By this means 27 putative LaeA orthologues from Eurotiomycetes, Dothidiomycetes and Sordariomycetes were identified (Table below). A phylogenetic analysis of these protein sequences produced a tree whose branching was consistent with the established phylogenetic relationship within these fungi, thus proving the orthology of the identified protein sequences. The T. reesei protein Trire2: 41617 was thus identified as the putative LaeA orthologue, LAE1.

| Genbank accession no. | Organism | Total score | coverage | E-value | identity [%] |
|---|---|---|---|---|---|
| EEU48790.1 | *Nectria haemato-cocca* | 255 | 89% | 4,00E-66 | 46 |
| XP 380833.1 | *Gibberella zeae* | 241 | 84% | 7,00E-62 | 45 |
| XP 001912425.1 | *Podospora anserina* | 204 | 85% | 1,00E-50 | 38 |
| XP 002837080.1 | *Tuber melanosporum* | 194 | 85% | 2,00E-47 | 36 |
| XP 001334837.1 | *Pyrenophora tritici-repentis* | 191 | 85% | 1,00E-46 | 35 |
| EEY21263.1 | *Verticillium albo-atrum* | 180 | 98% | 2,00E-43 | 35 |
| AAX68414.1 | *Aspergillus para-siticus* | 179 | 91% | 5,00E-43 | 36 |
| AAX68412.1 | *Aspergillus flavus* | 178 | 91% | 8,00E-43 | 36 |
| XP 002561699.1 | *Penicillium chry-sogenum* | 175 | 84% | 8,00E-42 | 36 |
| XP 752835.1 | *Aspergillus fumiga-*tus | 174 | 85% | 1,00E-41 | 36 |
| XP 960268.2 | *Neurospora crassa* | 134 | 84% | 2,00E-29 | 28 |

An examination of genomic and cDNA sequences revealed that the total coding region of Lae1 comprises 1061 nucleotides (SEQ ID No. 3) and is interrupted by three introns (62, 58 and 278 nts long, from 5' to 3'), giving rise to a 221 aa mature protein (SEQ ID No. 1) with a molecular weight of 25832 Da and an isoelectric point of 5.83 (data calculated by ProtPARAM; Gasteiger et al. 2005 (In) John M. Walker (ed): The Proteomics Protocols Handbook, Humana Press pp. 571-607 (2005)). It exhibited an overall amino acid identity of 28 and 24 % to the known proteins from A. nidulans and A. fumigatus, respectively. The protein contained the expected SAM domain, and four S and three T residues were detected which fulfill the consensus of phosphorylation by respective protein kinases (analyzed by NetPhos V 2.0; Blom et al. J. Mol. Biol. 294: 1351-1362 (1999)). Consistent with data from Aspergillus LaeA (Bok and Keller, Eukaryot. Cell 3, 527 (2004)), a conventional nuclear localization signal was not found.

Lae1 null mutants (Δlae1) were created by replacing the lae1 coding region with the orotidine-5-decarboxylase gene pyr410 in *T. reesei* KU70 (a ku70 delta strain). Growth of the Δlae1-deleted strain on simple carbon sources such as glycerol was similar to that of the parent strain, but growth on cellulose was severely impaired (Figure 1 a), indicating that the loss of lae1 function indeed leads to a defect in growth on cellulose. To test whether this is due to a loss of cellulase formation, two deletion mutants and the parent strain were cultivated on lactose, a carbon source which induces cellulase formation, but whose utilization is independent of cellulase formation. As shown in Figure 1b, growth of the parent strains and the mutants is indeed similar. However, significantly reduced cellulase activity was found in the mutant cultures (Figure 1 c). Consistent findings were obtained with xylan as a carbon source, on which the Δlae1-mutants exhibited a somewhat impaired growth, but xylanase activity was even more impaired (Figure 1 d).

The above data provided a first hint towards an effect of LAE1 on cellulase and hemicellulase formation in *T. reesei,* but the individual effects on the various cellulolytic and hemicel-lulolytic enzymes present in its genome cannot be deduced. In order to test whether the lae1 deletion indeed acts at the expression of its biomass degrading enzymes, a genome-wide approach was used: in total, 126 of the approximately 320 carbohydrate-active enzyme (CAZyme) genes of *T. reesei* are found in 25 discrete regions of the chromosome ranging from 14 kb to 275 kb in length. Thus, microarrays representing all 9130 unique alleles in the genome of *T. reesei* were used to examine their transcript levels when grown on lactose as a carbon source. 765 genes exhibited an at least twofold decrease in their hybridization intensity in the Δlae1 strain compared to QM 9414. Among these, 65 carbohydrate-active enzyme encoding genes were detected, which in majority comprised glycosyl hydrolases involved in cellulose and hemicellulose degradation (GHs; Table 1): they included all 10 cellulases (CEL5A, CEL5B, CEL6A, CEL7A, CEL7B, CEL12, CEL45, CEL61A, CEL61B and CEL74), both known swollenins (SWO1 SWO2; proteins carrying an expansin-like domain and that disrupt the crystalline cellulase structure) and CIPs (CIP1, CIP2; proteins that contain a signal peptide and a cellulose-binding domain), 5 of the 7 known β-glucosidase (CEL1A, CEL1B, CEL3C and CEL3D), and all 4 xylanases (XYN1-XYN4). The majority of the other affected GHs (21 of 28) comprised glycosidases active against various side chains in hemicelluloses.

**Table 1. Changes in carbohydrate-active enzyme gene expression in T. reesei by knocking out the function of lae1 * (sequences and protein ID number are obtainable at genome.jgi-psf.org/Trire2/Trire2.home.html; Martinez D et al., Nat. Biotechnol 26 (2008): 553-560)**

| | **Protein ID** | **downregulated** | **p-value** |
|---|---|---|---|
| ***Cellulases*** | | | |
| GH 5 endo-β-1,4-glucanase Cel5A | 120312 | 17,338 | 0,000896 |
| GH5 endo-β-1,4-glucanase CEL5B | 82616 | 5,179 | 0,00219 |
| GH6 Cellobiohydrolase 2 CEL6A | 72567 | 15,565 | 0,00106 |
| GH7 endo-β,4-glucanase EGL1 | 122081 | 3,019 | 0,00126 |
| GH7 cellobiohydrolase 1 CEL7A | 123989 | 6,841 | 0,000994 |
| GH12 endo-β-1,4-glucanase 12a | 123232 | 15,789 | 0,00132 |
| GH45 endo-β-1,4-glucanase EG5 | 49976 | 14,409 | 0,000892 |
| GH61 endo-β-1,4-glucanase CEL61A | 73643 | 25,659 | 0,000851 |
| GH61endo-β-1,4-glucanase CEL61B | 120961 | 40,524 | 0,000836 |
| GH1 β-glucosidase CEL1B | 22197 | 3,454 | 0,000918 |
| GH1 β-glucosidase CEL1A | 120749 | 2,428 | 0,00107 |
| GH3 β-glycosidase of uncertain specificity | 108671 | 2,833 | 0,00142 |
| GH3 β-glucosidase CEL3D | 46816 | 2,115 | 0,00525 |
| GH3 β-glucosidase CEL3C | 82227 | 3,555 | 0,00153 |
| ***nonenzymatic cellulose attacking enzymes*** | | | |
| CIP2 | 123940 | 3,232 | 0,00118 |
| CIP1 | 73638 | 16,794 | 0,000855 |
| CBM13 protein | 111094 | 8,529 | 0,00407 |
| swollenin | 123992 | 3,714 | 0,000847 |
| swollenin-like, 84 % ID to 123992 | 111874 | 6,17 | 0,00153 |
| ***xylanases*** | | | |
| GH10 xylanase XYN3 | 120229 | 4,392 | 0,00202 |
| GHllxylanase XYN1 | 74223 | 2,038 | 0,00213 |
| GH11 xylanase XYN2 | 123818 | 23,487 | 0,000931 |
| GH30 xylanase XYN4 | 111849 | 2,121 | 0,000885 |
| GH3 β-xylosidase BXL1 | 121127 | 17,003 | 0,000896 |
| GH43 β-xylosidase/α-arabinofuranosidase | 3739 | 2,752 | 0,000911 |
| GH74 xyloglucananase CEL74a | 49081 | 3,051 | 0,0009 |
| ***hemicellulose side chain cleaving enzymes*** | | | |
| CE5 acetyl xylan esterase AXE1 | 73632 | 6,821 | 0,000951 |
| GH67 α-glucuronidase AGU1 | 72526 | 13,365 | 0,00101 |
| GH62, α-L-arabinofuranosidase ABF2 | 76210 | 14,836 | 0,000893 |
| GH54, L-α-arabinofuranosidase ABF1 | 55319 | 2,201 | 0,00158 |
| GH95 α-fucosidase | 58802 | 9, 946 | 0,00544 |
| GH95 α-fucosidase | 5807 | 3, 606 | 0,0018 |
| GH92 α-1,2-mannosidase | 74198 | 6,097 | 0,000712 |
| GH92 α-1,2-mannosidase | 60635 | 2,154 | 0,00372 |
| GH47 α-1,2-mannosidase | 45717 | 4,496 | 0,00133 |
| GH2 β-mannosidase | 69245 | 5, 937 | 0,00181 |
| GH27 α-galactosidase AGL1 | 72632 | 5,777 | 0,00134 |
| GH27 α-galactosidase AGL3 | 27259 | 2,065 | 0,00641 |
| ***pectinases*** | | | |
| GH28 polygalacturonase | 103049 | 2,382 | 0,00831 |

| | | | |
|---|---|---|---|
| * values are given as means of two biological replica; "down-regulation" is given as -fold decrease compared to the parent strain. | | | |

The 25 carbohydrate-active enzyme clusters in the *T. reesei* genome contain an average five-fold increase in carbohydrate-active enzyme gene density compared to the expected density for randomly distributed genes. 765 of the total 9130 genes in the *T. reesei* genome to be at least 2-fold downregulated in the Δlae1 strain were identified, thus implying that at a random distribution one at every twelfth gene should be found. If the genes would however be clustered as calculated above, the average gene density of LAE1-affected genes should be around 2.5. To investigate this, the 765 identified genes on the *T. reesei* scaffolds were mapped and searched for potential clusters. Indeed, 28 areas on 21 scaffolds were found that exhibited a fourfold increase of gene density over the random distribution.

Clusters of expressed genes affected by lae1-loss of function in T. reesei

| ***scaffold*** | ***cluster predicted**** | ***found***** | ***genes in cluster*** | ***expressed genes*** | ***gene density****** | ***CAZys*** |
|---|---|---|---|---|---|---|
| 1 | yes | 618-690 | 72 | 23 | 3,13 | 5 |
| | yes | 112-135 | 23 | 9 | 2,55 | 3 |
| 2 | yes | 410-417 | 7 | 2 | 3,5 | 2 |
| 3 | no | 4-27 | 23 | 6 | 3,83 | 2 |
| | yes | 530-547 | 17 | 6 | 2,83 | 2 |
| 4 | no | 285-293 | 8 | 5 | 1,6 | 1 |
| 5 | no | 3-18 | 15 | 5 | 3 | 1 |
| | yes | 209-233 | 24 | 8 | 3 | 1 |
| 6 | no | 134-138 | 4 | 4 | 1 | 1 |
| 7 | no | 226-252 | 26 | 8 | 3,25 | 2 |
| | yes | 394-419 | 25 | 9 | 2,77 | 2 |
| 8 | yes | 167-192 | 25 | 7 | 3,51 | 3 |
| 10 | yes | 183-201 | 18 | 10 | 1,8 | 2 |
| 13 | yes | 16-34 | 18 | 5 | 3,6 | 1 |
| | no | 43-61 | 18 | 6 | 3 | 1 |
| 14 | no | 187-194 | 7 | 3 | 2,33 | 1 |
| 16 | no | 120-134 | 14 | 4 | 3,5 | 2 |
| 19 | yes | 62-97 | 35 | 10 | 3,5 | 1 |
| | yes | 157-189 | 32 | 12 | 2, 67 | 1 |
| 22 | no | 48-98 | 50 | 17 | 2, 94 | 1 |
| 27 | yes | 40-59 | 19 | 5 | 3,8 | 1 |
| 28 | no | 68-80 | 22 | 7 | 3, 15 | 1 |
| | no | 111-117 | 6 | 4 | 1,5 | 3 |
| 29 | yes | 91-102 | 11 | 7 | 1,57 | 2 |
| 30 | no | 4-19 | 15 | 4 | 3,75 | 1 |
| 31 | yes | 43-53 | 10 | 4 | 2,5 | 1 |
| 33 | yes | 29-42 | 13 | 6 | 2,16 | 1 |
| 44 | no | 4-15 | 11 | 5 | 1,2 | 1 |

| | | | | | | |
|---|---|---|---|---|---|---|
| * specifies that the expressed genes were found within the area of clusters proposed by Martinez et al. (Nature Biotechnol. 26, 553 (2008)). ** "found" means that a clustering of expressed genes was found, using the method outlined *** gene density is defined as the number of genes present in a proposed cluster, divided by the number of genes whose expression was affected at least 2-fold by the lae1 deletion | | | | | | |

32 of the 67 downregulated CAZyme genes were located within these clusters. Interestingly, 13 of these 29 clusters were found in areas not previously predicted.

In order to confirm the results from microarray analysis, cel7A and cel6A were used as cellulase model genes and their expression in the parent strain and in the Δlae1 strain was specifically tested by Real Time PCR. These cells were cultivated on lactose, sophorose (a disaccharide confering high cellulase induction in resting cells) and on the non-inducing carbon source glycerol (Figure 2). The data confirmed the finding of the microarray experiments as gene expression was absent in the Δlae1 strains on all three carbon sources. The results also demonstrate that the nature of the inducer does not influence the epigenetic regulation of cellulase formation.

Having identified LAE1 as a regulator of cellulase and hemicellulase biosynthesis in *T. reesei,* it was investigated whether an enhanced activity of LAE1 would even stimulate cellulase formation. To test this, two approaches were used: in one, a second copy of *T. reesei* lae1 was introduced into its genome.

In another one, lae1 downstream of the regulatory signals of the tef1 (elongation factor 1α-encoding) gene was fused which allow high constitutive expression. To this end, 740 bp of the promoter region of *H. jecorina tef1* (Genbank accession number Z23012.1) were amplified by PCR using the oligonucleotides tef1Xhofw and tef1ClaSalrv(Table A, above); the *XhoIlSalI-*restricted *tef1* fragment was then cloned in the corresponding sites of pLH1hph, which contains an E. *coli* hygromycin B phosphotransferase (hph) expression cassette as fungal selection marker. A 1,820-bp lael PCR fragment including the coding and terminator region was amplified with the oligonucleotides TrLae1ATGCla and TrLae1TermHind (Table A, above) and inserted downstream of the *tef1* promoter region at a *ClaI*/*HindIII* cleavage site resulting in vector Ptef1lae1hph.

Five and three *T. reesei* strains that contained a single lae1 copy and the *tef1:lae1* construct, respectively, integrated ectopically in its genome, were examined for their ability to form cellulases on lactose (Figure 4). Particularly the tef1:lae1 copies indeed exhibited an up to 10-fold increased cellulase formation and cel7A and cel6A gene expression, and this increased expression correlated with increased expression of lae1 in these strains.

The data presented clearly show that epigenetic manipulation significantly influences cellulase gene transcription in T. reesei, and thus represents a so far overlooked area for strain improvement by recombinant techniques. Since LAE1 acts by inactivating the heterochromatin protein HepA or the H3K9 methyltransferase Clr49 these findings further show that a loss-of-function in these genes also leads to an increase in cellulase formation. Orthologues for these two genes have been detected in *T. reesei.*

## Claims

1. Isolated polypeptide with transcription promoting protein methyltransferase activity having at least 80% identity with amino acid sequence SEQ ID No. 1.

2. Isolated nucleic acid molecule encoding an isolated polypeptide according to claim 1.

3. Isolated nucleic acid molecule according to claim 2, **characterised in that** the nucleic acid molecule exhibits at least 80% identity with nucleic acid sequence SEQ ID No. 2.

4. Vector comprising an isolated nucleic acid molecule according to claim 2 or 3.

5. Vector according to claim 4 further comprising at least one promoter operably linked to said nucleic acid molecule.

6. Vector according to claim 5, **characterised in that** the at least one promoter is selected from the group consisting of *tef1* promoter (transcription elongation factor 1), *gpg1* promoter, *pki1* promoter, enol promoter and *pgk1* promoter.

7. Recombinant host cell comprising a nucleic acid molecule according to claim 2 or 3 or a vector according to any one of claims 4 to 6.

8. Host cell according to claim 7, **characterised in that** said cell comprises more than one copy of the nucleic acid molecule according to claim 2 or 3 or the vector according to any one of claims 4 to 6.

9. Host cell according to claim 7 or 8, **characterised in that** said host cell is a fungus, preferably a fungus of the class of Sordariomycetes, more preferably a fungus of the family of Hypocreaceae, even more preferably a fungus of the genus of Trichoderma, in particular Trichoderma reesei.

10. Genetically modified Trichoderma reesei cell overexpressing a polypeptide with transcription promoting protein methyltransferase activity having at least 80% identity with amino acid sequence SEQ ID No. 1 compared to a wild-type Trichoderma reesei cell.

11. Trichoderma reesei cell according to claim 10, **characterised in that** the Trichoderma reesei cell comprises a vector according any one of claims 4 to 6.

12. Trichoderma reesei cell according to claim 10 or 11, **characterised in that** said cell comprises at least one mutation within the 5'-region of the genome location comprising nucleic acid sequence SEQ ID No. 2 or a variant thereof having at least 80% identity to SEQ ID No. 2.

13. Trichoderma reesei cell according to claim 12, **characterised in that** the at least one mutation is a deletion, an insertion or a point mutation.

14. Trichoderma reesei cell according to claim 12 or 13, **characterised in that** within the 5'-region of the genome location comprising nucleic acid sequence SEQ ID No. 2 a promoter, a transcription factor binding site or a functional fragment thereof is inserted resulting in an increased expression of the polypeptide encoded by the nucleic acid sequence SEQ ID No. 2 or a variant thereof having at least 80% identity to SEQ ID No. 2 compared to the wild-type Trichoderma reesei.

15. Trichoderma reesei cell according to any one of claims 10 to 14, **characterised in that** the Trichoderma reesei cell comprises further a recombinant nucleic acid coding region operatively linked to a Trichoderma reesei promoter sequence and/or a vector comprising a nucleic acid coding region operatively linked to a Trichoderma reesei promoter sequence, wherein the nucleic acid coding region is under the transcriptional control of said promoter sequence.

16. Trichoderma reesei cell according to claim 15, **characterised in that** the nucleic acid coding region encodes for a peptide, a polypeptide, a protein or a functional DNA or RNA.

17. Trichoderma reesei cell according to claim 15 or 16, **characterised in that** the nucleic acid coding region is introduced into the genome of said Trichoderma reesei cell by gene replacement at the genome location comprising the coding regions for GH 5 endo-β-1,4-glucanase Cel5A (protein ID 120312), GH5 endo-β-1,4-glucanase CEL5B (protein ID 82616), GH6 Cellobiohydrolase 2 CEL6Ac (protein ID 72567), GH7 endo-β,4-glucanase EGL1 (protein ID 122081), GH7 cellobiohydrolase 1 CEL7A (protein ID 123989), GH12 endo-β-1,4-glucanase 12a (protein ID 123232), GH45 endo-β-1,4-glucanase EG5 (protein ID 49976), GH61 endo-β-1,4-glucanase CEL61A (protein ID 73643), GH61endo-β-1,4-glucanase CEL61B (protein ID 120961), GH1 β-glucosidase CEL1B (protein ID 22197), GH1 β-glucosidase CEL1A (protein ID 120749), GH3 β-glycosidase of uncertain specificity (protein ID 108671), GH3 β-glucosidase CEL3D (protein ID 46816), GH3 β-glucosidase CEL3C (protein ID 82227), CIP2 (protein ID 123940), CIP1 (protein ID 73638), CBM13 protein (protein ID 111094), swollenin (protein ID 123992), swollenin-like, 84 % ID to 123992 (protein ID 111874), GH10 xylanase XYN3 (protein ID 120229), GHllxylanase XYN1 (protein ID 74223), GH11 xylanase XYN2 (protein ID 123818), GH30 xylanase XYN4 (protein ID 111849), GH3 β-xylosidase BXL1 (protein ID 121127), GH43 β-xylosidase/α-arabinofuranosidase (protein ID 3739), GH74 xyloglucananase CEL74a (protein ID 49081), CE5 acetyl xylan esterase AXE1 (protein ID 73632), GH67 α-glucuronidase AGU1 (protein ID 72526), GH62, α-L-arabinofuranosidase ABF2 (protein ID 76210), GH54, L-α-arabinofuranosidase ABF1 (protein ID 55319), GH95 α-fucosidase (protein ID 58802), GH95 α-fucosidase (protein ID 5807), GH92 α-1,2-mannosidase (protein ID 74198), GH92 α-1,2-mannosidase (protein ID 60635), GH47 α-1,2-mannosidase (protein ID 45717), GH2 β-mannosidase (protein ID 69245), GH27 α-galactosidase AGL1 (protein ID 72632), GH27 α-galactosidase AGL3 (protein ID 27259) and/or GH28 polygalacturonase (protein ID 103049).

18. Method for the recombinant production of a peptide, a polypeptide, a protein or a functional DNA or RNA comprising the step of cultivating a genetically modified Trichoderma reesei cell according to any one of claims 10 to 17.
